# EUROPEAN PATENT SPECIFICATION

(11) **EP 1 986 575 B1**
(45) Date of publication and mention of the grant of the patent: **21.09.2011**
(21) Application number: 07705623.2
(22) Date of filing: 19.02.2007
(51) Int. Cl.: A61F 2/40

(54) **A GLENOID COMPONENT OF A SHOULDER JOINT PROSTHESIS**
SCHULTERGELENKKOMPONENTE EINER SCHULTERGELENKPROTHESE
COMPOSANT GLENOIDE D'UNE PROTHESE D'ARTICULATION DE L'EPAULE

(30) Priority: 23.02.2006 GB 0603572
(43) Date of publication of application: 05.11.2008
(60) Divisional application: 10170058.1
(73) Proprietor: DePuy (Ireland), Ringaskiddy (IE)
(72) Inventor: DEWILDE, Lieven, 9000 Gent (BE); KARELSE, Anne, 9190 Stekene (BE)
(74) Representative: Belcher, Simon James
(86) International application number: PCT/IB2007/000404
(87) International publication number: WO 2007/096741

(56) References cited:
- EP-A- 1 520 561
- FR-A- 2 825 263
- FR-A1- 2 843 293

## Description

This invention relates to a glenoid component of a shoulder joint prosthesis.

A glenoid component of a shoulder joint prosthesis is used in the repair and replacement of a diseased or damaged shoulder joint. The glenoid component is fastened in the glenoid region to the scapula and provides a surface with which a humeral implant contacts and articulates.

One type of common glenoid implant is a "metal backed" glenoid implant. A metal backed glenoid implant comprises a frame part for attachment to the glenoid region of the scapula, and a body part which provides the surface which the humeral implant contacts and articulates. As will be understood, if the metal backed glenoid implant is part of an anatomic shoulder prosthesis, then the body part will be a "cup" which presents a rounded concave surface. However, if the metal backed glenoid implant is part of a reverse shoulder prosthesis, then the body part will be a "head" which presents a rounded convex surface.

It is important that the frame part can be secured to the scapula sufficiently strongly to withstand forces exerted on the glenoid implant. During implantation, the location of the frame part relative to the glenoid is generally selected so that the joint prosthesis matches as closely as possible the anatomical configuration of the natural joint. Accordingly, it can be desirable to prevent any movement of the frame relative to the glenoid once implanted. Also, an improperly anchored frame can result in additional damage being caused to the scapula.

FR-2843293 discloses a shoulder joint implant which comprises a metallic base which can be fixed to a patient's scapula. The base receives a polyethylene insert. The base has a profile and fittings which can engage complementary fittings on the insert enabling the two to be connected with a desired angular orientation. The base can be fastened to the scapula using bone screws which converge as they extend from the base into the scapula.

The present invention provides a frame part of a glenoid component which provides at least two bores, each which are configured so that the distance measured between bone screws received therethrough decreases with increasing distance away from the frame.

Accordingly, the invention provides a glenoid component of a shoulder joint prosthesis for fixation to the glenoid region of a scapula, as defined in claim 1.

It has been found that a frame fastened to the glenoid region of a scapula by bone screws that converge toward each other away from the medial surface of the frame ("converging screws"), can improve the anchorage of the frame on the scapula. The use of converging screws can increase the chance of the screws becoming anchored within structurally sound portions of the scapula. In particular, the screws can be directed into the lateral border pillar and the spine pillar of the scapula. The problem of bone screws being improperly and/or insufficiently anchored is particularly an issue during revision of a glenoid implant. Each implantation requires the creation of holes in the scapula, for example to receive bone screws and/or pegs. Accordingly, achieving adequate anchorage of a frame during revision of a glenoid implant can be difficult due to the deterioration in the structural integrity of the bone, caused by the presence of a number of holes. It has been found that the anchorage of frames of revised glenoid implants can be improved by using converging screws.

The frame of the present invention has the advantage that screws received through its bores are guided by the bores so that the screws converge toward each other as the distance from the medial surface of the frame increases. Accordingly, the frame provides the advantages associated with anchoring a frame to a scapula using converging screws discussed above.

The cross-sectional shape of the frame can be any regular or irregular shape. For example, the cross-sectional shape of the frame can be rectangular, or hexagonal. Preferably, the cross-sectional shape of the frame is generally rounded, for example oval or generally elliptical, especially generally circular. A rounded frame can best match the anatomical shape of the glenoid region.

Preferably, the frame has a substantially planar configuration. However, the medial and/or lateral surfaces need not necessarily be substantially planar. For example, it can be preferable for the medial surface of the frame to be slightly convex. This can help to ensure that when implanted, the medial surface of the frame fits snugly against the concave configuration of the scapula in the glenoid region.

Preferably, the frame has a generally planar configuration and the bores are configured so that the distance between the axes of the bores, measured along each of two orthogonal measurement axes in the plane of the frame, decreases in a direction away from the medial surface of the frame, in which one of the measurement axes and the axis of the one of the bores lie in a common plane. This has been found to improve the anchorage of the frame within the scapula.

The bores of the frame are configured so that the axes of the bores do not fall within a common plane. The transverse distance measured between the axes decreases as the distance away from the medial surface of the frame towards the scapula increases, at least initially.

Only one of the axes of the bores needs to be inclined relative to a first plane that extends perpendicular to the plane of the frame to ensure that the axes of the bores converge.

Preferably, the axis of the first bore and the axis of the second bore are both inclined relative to the first plane. This can be advantageous as it can improve the anchorage of the frame in the scapula.

Preferably, the axis of the first bore is inclined relative to the first plane by at least 5°, more preferably by at least 10°, especially preferably by at least 20°, most preferably by 24°. Preferably, the axis of the first bore is inclined relative to the first plane by not more than 35°, more preferably by not more than 30°, especially by not more than 25°.

Preferably, the axis of the second bore is inclined relative to the first plane by at least 10°, more preferably by at least 20°, especially preferably by at least 30°, most preferably by 33 °. Preferably, the axis of the second bore is inclined relative to the first plane by not more than 50°, more preferably by not more than 40°, especially preferably by not more than 35°.

Preferably, the frame has a generally planar configuration and in which the bores are configured so that the distance between the axes of the bores, measured along each of two orthogonal measurement axes in the plane of the frame, decreases in a direction away from the medial surface of the frame, in which one of the measurement axes and the axis of one of the bores lie in a common plane. This has been found to improve the anchorage of the frame to the scapula.

Preferably, the axis of the first bore is inclined relative to first and second mutually perpendicular planes, the first and second planes being perpendicular to the plane of the frame when the frame is generally planar. Preferably, the axis of the second bore is inclined relative to the first and second mutually perpendicular planes, the first and second planes being perpendicular to the plane of the frame when the frame is generally planar. It has been found that this can improve the anchorage of the frame to the scapula. The minimum distance in both dimensions will be greater than 0 mm. The minimum distance in both dimensions will be sufficient for each screw to be received through the bores without colliding with other screws.

Preferably, the axis of the first bore is inclined relative to the second plane by at least 5°, more preferably by more than 7°, especially preferably by at least 10°, most preferably by 12°. Preferably, the axis of the first bore is inclined relative to the second plane by not more than 20°, more preferably by not more than 17°, especially preferably by not more than 15°.

Preferably, the axis of the second bore is inclined relative to the second plane by at least 1 °, more preferably by at least 2°, most preferably by 3 °. Preferably the axis of the second bore is inclined relative to the second plane by not more than 10°, more preferably by not more than 5°, especially preferably by not more than 4°.

Preferably, the angle between (a) a first line extending between the centre point of the first bore and the centre point of the frame, and (b) a second line extending between the centre point of the second bore and the centre point of the frame, is less than 180°. Such configuration of the bores can improve the anchorage of the frame to the scapula. Preferably, the angle between (a) and (b) is not more than 155°, especially preferably not more than 150°, most preferably 145°. Preferably, the angle between (a) and (b) is at least 100°, more preferably at least 120°, especially preferably at least 130°, most preferably at least 140°.

Preferably, the distance between the centre point of the first bore and the centre point of the frame, is at least 5 mm, more preferably at least 6 mm, especially preferably at least 7 mm, most preferably approximately 8 mm. Preferably, the distance between the centre point of the first bore and the centre point of the frame is not more than 11 mm, more preferably not more than 10 mm, especially preferably not more 9 mm.

Preferably, the distance between the centre point of the first bore and the centre point of the frame, is the same as the distance between the centre point of the second bore and the centre point of the frame.

Preferably, the bores of the frame are countersunk so that the heads of screws received therethrough do not protrude above the lateral surface. This can help prevent the heads of the screws interfering with the body part when the body is received on the frame.

The countersunk portion of the bores can be configured such that the head of a screw received through the bore can only be properly seated in the countersunk portion in one angular orientation. For example, the cross-sectional shape of the countersunk portion, in a plane perpendicular to the diameter of the bore can be square in shape. The countersunk portion can be configured such that when the head of the screw is properly seated in the countersunk portion, the axis of the screw is coaxial with the axis of the bore.

Preferably, the cross-sectional shape of the countersunk portion, in a plane perpendicular to the diameter of the bore, is such that a screw having a correspondingly shaped head can be properly seated in the bore in more than one angular orientation. The bores and the screws will be configured such that the screws can only be properly received in the frame when the screws are converged. Preferably, the cross-sectional shape is rounded so that the screw can be properly seated through a range of angles. This can be advantageous as it can allow for slight variations in the degree by which the screws converge and can give the surgeon some degree of freedom in choosing the location of the screws in the scapula. This can remove the need to have different shaped and sized frame parts for different patients having different shaped and sized scapulas. Preferably, the axis of a screw received through a bore can not be inclined by more than 10 relative to the axis of the bore, more preferably not by more than 5°.

The diameter of the bores are sufficiently large so as to receive bone screws therethrough. The diameter of the bores can be configured such that the bone screws received therethrough can only be received in one angular orientation. Preferably, the diameter of the bores are slightly larger than the diameter of the shaft of the bone screws so that the bone screws can be received therethrough in a range of angular orientations.

The frame will generally be made from metallic based materials which are conventionally used in the manufacture of surgical implants. Certain stainless steels can be particularly preferred. Other materials include titanium and titanium alloys.

Implant components provided by the invention can be used in a method of shoulder joint replacement which comprises fastening a frame part of a glenoid component for a shoulder joint prosthesis to the scapula in the glenoid region thereof using bone screws which extend through the frame part into the lateral border pillar and the spine pillar of the scapula.

The method can include surgical planning steps, in which images are generated of the patient's glenoid to allow areas of high bone density to be identified. The information generated in from the images can enable the surgeon to position the screws visually with reference to visible landmarks. The images can also be used in a computer assisted surgical procedure, in which the positions of instruments used in the procedure are tracked relative to stored image data. The positions and orientations of the fixation screws can be identified relative to the bone tissue using the image data. The image data can be generated using techniques such as x-ray, computer tomography and magnetic resonance.

Accordingly, implant components provided by the invention can be used in a method of planning a shoulder joint replacement surgical procedure which comprises:
a. obtaining scan image data of the patient's scapula,
b. identifying paths for fixation screws for a component of a shoulder joint prosthesis extending into the lateral border pillar and the spine pillar of the scapula.

Preferably, the screws are arranged so that the distance measured between their respective axes decreases with increasing distance away from the medial surface of the frame towards the scapula.

Preferably, the method includes the step of locating a portion of the surface of the scapula which is generally circular and generally planar, and mounting the frame part of the glenoid component on that portion of the scapula surface.

Embodiments of the present invention will now be described by way of example only with reference to the accompanying drawings in which:
Figure 1 is a schematic side view of a glenoid component for the right hand side of the body, comprising a frame part according to the present invention, fastened to the glenoid region of a scapula;
Figure 2 is a schematic perspective view of the frame part shown in Figure 1, from a first angular orientation;
Figure 3 is a schematic perspective view of the frame part shown in Figure 1, from a second angular orientation;
Figure 4 is a schematic side view of the frame part shown in Figure 1;
Figure 5 is a schematic plan view of the frame part shown in Figure 1; and
Figure 6 is a schematic side view of the frame part of the glenoid component shown in Figure 1, fastened to the glenoid region of the scapula.

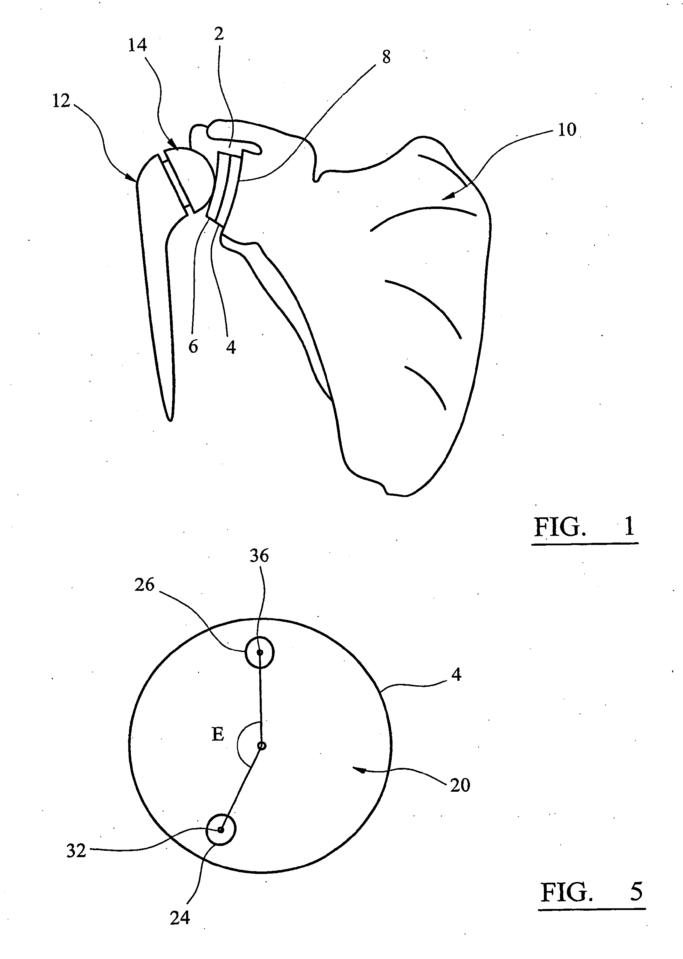

Referring to the drawings, Figure 1 shows a glenoid component 2 of a shoulder joint prosthesis, implanted in the glenoid region 8 of a scapula 10. Also shown is a humeral component 12 of the shoulder joint prosthesis, implanted in a humerus (not shown). The head 14 of the humeral component 12 is configured to articulate with the glenoid component 2.

The glenoid component 2, comprises a frame part 4 and a body part 6. The body part 6 is fastened to the frame part 4, which in turn is secured to the glenoid region 8 of the scapula 10 by bone screws 16, 18 (not shown in figure 1). The head 14 of the humeral implant articulates with the surface of the body part 6 that is lateral relative to the scapula (i.e. the "lateral surface") when the implant 2 is implanted. The lateral surface of the body part is slightly concave.

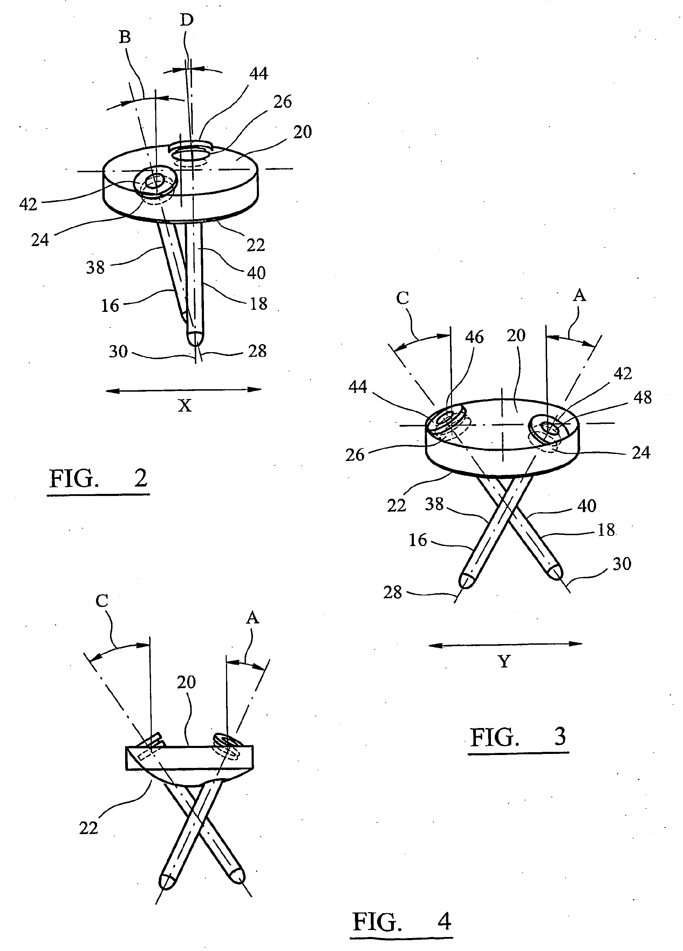

Figures 2 to 4 show the frame part 4 in more detail. The frame part 4 comprises a lateral surface 20 relative to the scapula 10 when the frame is implanted, (i.e. a "lateral surface"), and a medial surface 22 relative to the scapula 10 when the frame is implanted (i.e. a "medial surface"). As best shown in figure 4, the lateral surface 20 is planar, and the medial surface 22 is convex and rounded so as to match the anatomical configuration of the glenoid region 8. The frame has a generally planar configuration, the plane of the frame being parallel to the frame of the lateral surface 20.

The frame 4 further comprises first 24 and second 26 bores that extend between the lateral and medial surfaces for receiving the first 16 and second 18 bone screws. The bores are configured so that their axes 28, 30 are converged. As best shown in figures 2 and 3, the distance between the axes 28, 30, measured along each of two orthogonal measurement axes X and Y, in the plane of the frame, decreases as in a direction away from the medial surface 22 of the frame 4, and reaches a minimum distance along both measurement axes before diverging. The point along axes at which the distance between them is at a minimum is different along the X measurement axis than it is along the Y measurement axes.

As shown, the first bore 24 is configured so that its axis 28 is inclined relative to first and second mutually perpendicular planes, the first and second planes being perpendicular to the plane of the frame. As shown in Figure 4, the axis of the first bore 24 is inclined relative to the first plane by 24° (shown as angle A in Figure 4). The axis of the first bore 24 is inclined relative to the second plane by 12° (shown as angle B in Figure 3).

The second bore 26 is also configured so that its axis 30 is inclined relative to the same first and second mutually perpendicular planes. As shown in Figure 4, the axis of the second bore 26 is inclined relative to the first plane by 33° (shown as angle C in Figure 4). The axis of the second bore 26 is inclined relative to the second plane by 3° (shown as angle D in Figure 3).

As shown in Figure 5, the first 24 and second 26 bores are arranged within the frame such that the angle E between a first line extending between the centre point 32 of the first bore 24 and the centre point 34 of the frame 4, and a second line extending between the centre point 36 of the second bore 26 and the centre point of the frame, is 145°.

In the embodiment shown, the bores 24, 26 are configured so that the shank portions 38, 40 of the screws 16, 18 can be slidingly received therethrough. The diameter of the head portions 42, 44 of the screws are larger than the diameters of the bores, and therefore cannot be slide through the bores. The head portions 42, 44 of the screws have non-circular recesses 46, 48 formed therein for receiving a tool (not shown) for imparting a rotational force on the screws. In the embodiment shown, the bores 24, 26 are only partially countersunk. Therefore, a portion of the head portions 42, 44 of the screws protrude from the lateral surface 20 of the frame 4.

Due to the screws received through the bores of a frame according to the present invention being angularly inclined relative to the plane of the frame, it can be necessary in some circumstances to use screws longer than 18 mm to achieve sufficient anchorage within the scapula. Typically, the length of the bone screws 16, 18 will be at least about 18 mm, preferably at least about 24 mm, for example at least about 30 mm. The length will often be not more than about 120 mm, preferably not more than about 80 mm, more preferably not more than about 50 mm.

In use, the glenoid region 8 of the scapula 10 is prepared before implanting the glenoid implant 2. This includes preparing holes suitable for receiving the bone screws 16, 18 of the implant 2. Such holes can be prepared by using a suitable drilling tool and drilling guide. In some embodiments, the frame part 4 can be configured to enable the drilling guide to be fastened to the frame part 4, so that the drill guide and a bore of the frame are coaxial. Accordingly, in this way, the bore is also used as a drill guide. This can help ensure that the axis of the drilled hole in the bone is coaxial with the axis of the bore of the frame through which a screw will be received. However, it is also envisaged that the locations and orientations of the bone screw holes will be capable of being identified by the surgeon with reference to pre-operative images of the patient's anatomy and to landmarks on the anatomy which are identified in the images.

Once the glenoid region 8 and the holes for the bone screws 16, 18 are prepared, the frame 4 is secured to the scapula. The frame 4 is located on the glenoid region and the screws 16, 18 are passed through the frame 4 and screwed into the holes in the scapula. Once securely anchored, the body part 6 can be fastened to the frame part. Design features which can be used to fasten the body part to the frame part are known from existing orthopaedic prostheses which are constructed from separate modular parts, and include for example threaded fasteners, cooperating taper surfaces which fit together with an interference fit ("Morse taper"), etc.

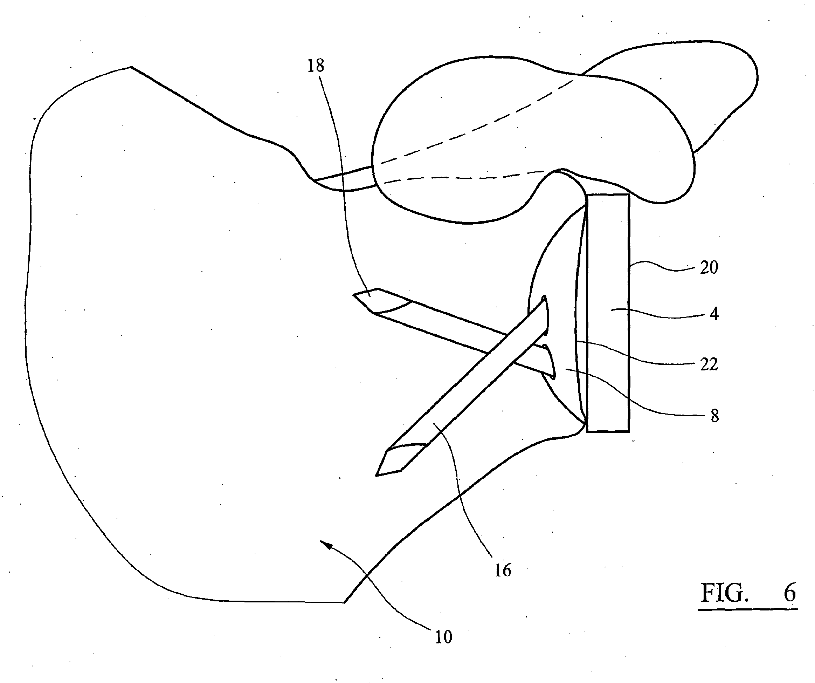

Figure 6 illustrates the frame part 4 secured to the scapula 10 by the first 16 and second 18 screws which extend into the lateral border pillar and the spine pillar of the scapula respectively.

## Claims

1. A glenoid component (2) of a shoulder joint prosthesis for fixation to the glenoid region (8) of a scapula (10), the component comprising a body part (6) of the prosthesis which presents a surface for articulation with the corresponding articulating surface of a humeral component, and a frame part (4) which has:
a lateral surface (20) on which the body part can be mounted,
a medial surface (22) generally opposite the lateral surface, and
first and second bores (24, 26) that extend between the lateral and medial surfaces for receiving screws (16, 18) so as to fasten the frame part to the scapula, in which the bores are configured so that their axes (28, 30) converge, so that the distance measured between the axes decreases initially with increasing distance away from the medial surface of the frame towards the scapula,
**characterised in that** the axes of the bores do not fall within a common plane.

2. A glenoid component as claimed in claim 1, in which the frame (4) has a generally circular configuration.

3. A glenoid component as claimed in claim 2, in which the bores (24, 26) are located inside the circular boundary of the frame (4).

4. A glenoid component as claimed in claim 1, in which the frame (4) has a generally planar configuration, and in which the axis (28) of the first bore (24) is inclined relative to first and second mutually perpendicular planes, the first and second planes being perpendicular to the plane of the frame.

5. A glenoid component as claimed in claim 4, in which the axis (30) of the second bore (26) is inclined relative to the said first and second planes.

6. A glenoid component as claimed in claim 4, in which the one of the first or second planes extends through the centre point of one of the first or second bores (24, 26).

7. A glenoid component as claimed in claim 4, in which the second plane extends through the centre point of the second bore (26). ,

8. A glenoid component as claimed in claim 1, in which the angle between a first line extending between the centre point of the first bore (24) and the centre point of the frame part (4), and a second line extending between the centre point of the second bore (26) and the centre point of the frame part, is less than 180°.

9. A glenoid component as claimed in claim 1, in which the bores (24, 26) are countersunk so that the heads of screws (16, 28) received therethrough do not protrude above the lateral surface.

10. A glenoid component as claimed in claim 9, in which the cross-sectional shape of the countersunk portion of the bore, in a plane perpendicular to the diameter of the bore, is such that a screw having a correspondingly shaped head can,be properly seated in the bore in more than one angular orientation.

11. A glenoid component as claimed in claim 1, in which the articulating surface of the body part(6) is convex for articulation with the articulating surface of a humeral component of a joint prosthesis which is concave.

12. A glenoid component as claimed in claim 1, in which the articulating surface of the body part (6) is concave for articulation with the articulating surface of a humeral component of a joint prosthesis which is convex.

13. A shoulder joint prosthesis which comprises a glenoid component (2) as claimed in claim 1, and a humeral component.

## Patentansprüche

1. Schultergelenkkomponente (2) einer Schultergelenkprothese für das Festlegen am Schultergelenkbereich (8) eines Schulterblattes (10), wobei die Komponente einen Körperteil (6) der Prothese, die eine Fläche für die Gelenkbildung mit der entsprechenden gelenkbildenden Fläche einer Oberarmkomponente darstellt, und einen Rahmenteil (4) aufweist, der:
eine laterale Fläche (20), auf der der Körperteil angebracht werden kann,
eine mediale Fläche (22) im Allgemeinen der lateralen Fläche gegenüberliegend, und eine erste und eine zweite Bohrung (24, 26), die sich zwischen der lateralen und der medialen Fläche erstreckt, zum Aufnehmen von Schrauben (16, 18), um so den Rahmenteil an dem Schulterblatt zu befestigen, hat, wobei die Bohrungen so ausgelegt sind, dass ihre Achsen (28, 30) konvergieren, so dass der Abstand, der zwischen den Achsen gemessen wird, anfänglich mit zunehmender Entfernung weg von der medialen Fläche des Rahmens auf das Schulterblatt zu abnimmt,
**dadurch gekennzeichnet, dass** die Achsen der Bohrungen nicht in eine gemeinsame Ebene fallen.

2. Schultergelenkkomponente nach Anspruch 1, bei der der Rahmen (4) eine im Allgemeinen kreisförmige Ausgestaltung hat.

3. Schultergelenkkomponente nach Anspruch 2, bei der die Bohrungen (24, 26) sich innerhalb der kreisförmigen Grenze des Rahmens (4) befmden.

4. Schultergelenkkomponente nach Anspruch 1, bei der der Rahmen (4) eine im Allgemeinen planare Ausgestaltung hat und bei der die Achse (28) der ersten Bohrung (24) im Bezug auf eine erste und eine zweite angeordnete Ebene, die wechselseitig zueinander senkrecht sind, geneigt ist, wobei die erste und die zweite Ebene senkrecht zu der Ebene des Rahmens sind.

5. Schultergelenkkomponente nach Anspruch 4, bei der die Achse (30) der zweiten Bohrung (26) in Bezug auf die erste und die zweite Ebene geneigt ist.

6. Schultergelenkkomponente nach Anspruch 4, bei der sich eine, die erste oder die zweite Ebene, durch den Mittelpunkt einer, der ersten oder der zweiten Bohrung (24, 26), erstreckt.

7. Schultergelenkkomponente nach Anspruch 4, bei der sich die zweite Ebene durch den Mittelpunkt der zweiten Bohrung (26) erstreckt.

8. Schultergelenkkomponente nach Anspruch 1, bei der der Winkel zwischen einer ersten Linie, die sich zwischen dem Mittelpunkt der ersten Bohrung (24) und dem Mittelpunkt des Rahmenteils (4) erstreckt, und einer zweiten Linie, die sich zwischen dem Mittelpunkt der zweiten Bohrung (26) und dem Mittelpunkt des Rahmenteils erstreckt, kleiner als 180° ist.

9. Schultergelenkkomponente nach Anspruch 1, bei der die Bohrungen (24, 26) gesenkt sind, so dass die Köpfe von Schrauben (16, 28), die durch diese aufgenommen werden, nicht über die laterale Fläche hervorstehen.

10. Schultergelenkkomponente nach Anspruch 9, bei der die Querschnittsform des gesenkten Bereiches der Bohrung in einer Ebene senkrecht zu dem Durchmesser der Bohrung derart ist, dass eine Schraube mit einem entsprechend geformten Kopf in mehr als einer Winkelausrichtung richtig in die Bohrung eingesetzt werden kann

11. Schultergelenkkomponente nach Anspruch 1, bei der die gelenkbildende Fläche des Körperteiles (6) konvex ist, für die Gelenkbildung mit der gelenkbildenden Fläche einer Oberarmkomponente einer Gelenkprothese, die konkav ist.

12. Schultergelenkkomponente nach Anspruch 1, bei der die gelenkbildende Fläche des Körperteiles (6) konkav ist, für die Gelenkbildung mit der gelenkbildenden Fläche einer Oberarmkomponente einer Gelenkprothese, die konvex ist.

13. Schultergelenkprothese, die eine Schultergelenkkomponente (2) nach Anspruch 1 und eine Oberarmkomponente aufweist.

## Revendications

1. Composant glénoïde (2) d'une prothèse d'articulation de l'épaule destiné à être fixé à la région glénoïde (8) d'une omoplate (10), le composant comprenant une partie de corps (6) de la prothèse qui présente une surface destinée à s'articuler avec la surface articulée correspondante d'un composant huméral, et une partie de cadre (4) qui possède :
➢ une surface latérale (20) sur laquelle peut être montée la partie de corps,
➢ une surface médiale (22) généralement opposée à la surface latérale, et
➢ des premier et second alésages (24, 26) qui s'étendent entre la surface latérale et la surface médiale pour recevoir des vis (16, 18) de sorte à attacher la partie de cadre à l'omoplate, les alésages étant configurés de telle sorte que leurs axes (28, 30) convergent, de telle sorte que la distance mesurée entre les axes diminue initialement avec l'augmentation de la distance à partir de la surface médiale du cadre vers l'omoplate,
**caractérisé en ce que** les axes des alésages ne se trouvent pas à l'intérieur d'un plan commun.

2. Composant glénoïde selon la revendication 1, dans lequel le cadre (4) a une configuration généralement circulaire.

3. Composant glénoïde selon la revendication 2, dans lequel les alésages (24, 26) se trouvent à l'intérieur de la limite circulaire du cadre (4).

4. Composant glénoïde selon la revendication 1, dans lequel le cadre (4) a une configuration généralement planaire, et dans lequel l'axe (28) du premier alésage (24) est incliné par rapport à des premier et second plans mutuellement perpendiculaires, les premier et second plans étant perpendiculaires au plan du cadre.

5. Composant glénoïde selon la revendication 4, dans lequel l'axe (30) du second alésage (26) est incliné par rapport auxdits premier et second plans.

6. Composant glénoïde selon la revendication 4, dans lequel le premier plan ou le second plan s'étend à travers le point central de l'un du premier alésage ou du second alésage (24, 26).

7. Composant glénoïde selon la revendication 4, dans lequel le second plan s'étend à travers le point central du second alésage (26).

8. Composant glénoïde selon la revendication 1, dans lequel l'angle entre une première ligne s'étendant entre le point central du premier alésage (24) et le point central de la partie de cadre (4), et une seconde ligne s'étendant entre le point central du second alésage (26) et le point central de la partie de cadre, est inférieur à 180°.

9. Composant glénoïde selon la revendication 1, dans lequel les alésages (24, 26) sont fraisés de telle sorte que les têtes des vis (16, 28) reçues à travers ceux-ci ne fassent pas saillie au-dessus de la surface latérale.

10. Composant glénoïde selon la revendication 9, dans lequel la forme en coupe transversale de la partie fraisée de l'alésage, dans un plan perpendiculaire au diamètre de l'alésage, est telle qu'une vis ayant une tête façonnée en correspondance peut être correctement logée dans l'alésage dans plusieurs orientations angulaires.

11. Composant glénoïde selon la revendication 1, dans lequel la surface articulée de la partie de corps (6) est convexe pour s'articuler avec la surface articulée d'un composant huméral d'une prothèse d'articulation qui est concave.

12. Composant glénoïde selon la revendication 1, dans lequel la surface articulée de la partie de corps (6) est concave pour s'articuler avec la surface articulée d'un composant huméral d'une prothèse d'articulation qui est convexe.

13. Prothèse d'articulation de l'épaule qui comprend un composant glénoïde (2) selon la revendication 1, et un composant huméral,
